# EUROPEAN PATENT APPLICATION

(11) **EP 4 659 657 A1**
(43) Date of publication of application: **10.12.2025**
(21) Application number: 24211736.4
(22) Date of filing: 08.11.2024
(51) Int. Cl.: A61B 5/00, A61B 8/00, H01S 5/026

(54) **HANDHELD DEVICE AND SYSTEM FOR OPTOACOUSTIC SENSING, IN PARTICULAR IMAGING**

(30) Priority: 07.06.2024 EP 24180941
(71) Applicant: iThera Medical GmbH, 81379 München (DE)
(72) Inventor: Eliason, Braden, 80538 München (DE); Zahnd, Dr. Guillaume, 81379 München (DE); Longo, Dr. Antonia, 80538 München (DE); Leisching, Dr. Patrick, 81247 München (DE); Konradl, Josef, 85235 Odelzhausen (DE)
(74) Representative: Wallinger Ricker Schlotter Tostmann

(57) **Abstract**

The disclosure relates to a handheld device for optoacoustic imaging of an object, wherein the device comprises: i) at least one VCSEL sensor unit comprising a vertical-cavity surface-emitting laser (VCSEL), a laser resonator and a photodiode provided in and/or at the laser resonator and being arranged and/or configured to illuminate the object with first electromagnetic radiation emerging from the laser resonator, allow second electromagnetic radiation, which has been reflected and/or scattered by the object in response to illuminating the object with the first electromagnetic radiation, to enter the laser resonator and mix and/or interfere with the first electromagnetic radiation, whereby an interference signal is obtained, and detect the interference signal with the photodiode, wherein the at least one electronic controller is configured to derive, based on the detected interference signal, positional information regarding a position and/or distance and/or movement of the at least one VCSEL sensor unit and/or the handheld device relative to the object; and/or ii) at least one vertical-cavity surface-emitting laser (VCSEL) configured to illuminate the object with first electromagnetic radiation, a detector, in particular an optical or ultrasound detector, configured to, in particular incoherently, detect a response signal, in particular an optical signal or an ultrasound signal, emanating from the object in response to illuminating the object with the first electromagnetic radiation, wherein the at least one electronic controller is configured to derive, based on the detected response signal, a time measure characterizing a duration (time of flight) it takes for the first electromagnetic radiation to travel from the VCSEL to the object and, after reflection or conversion into an ultrasound wave by the object, back to the detector, and to derive positional information regarding a height position and/or distance of the at least one VCSEL and/or handheld device relative to the object. The disclosure further relates to an according handheld device for ultrasound imaging and an according system for optoacoustic or ultrasound imaging.

## Description

The present disclosure relates to a handheld device and system for optoacoustic sensing, in particular imaging, of an object according to the independent claims. The present disclosure further relates to a corresponding handheld device and system for ultrasound sensing, in particular imaging.

Currently, routine clinical use and Point-of-Care (PoC) optoacoustic imaging (herein also referred to as "OA" or "OAI") devices are non-existent because current scanner technology is bulky, expensive, power consuming (>1000 W) and complicated to operate. The state-of-the-art laser sub-component (including electronics) amounts for circa 50% of the total cost, size and power consumption. Laser technologies based on high power lasers (e.g., Nd:YAG+OPO), or LEDs have a low wall-plug efficiency and therefore require active cooling, consume a vast amount of energy, are bulky, and make the examination room hot and noisy (air conditioning needed). YAG-OPO or laser diodes as point source necessitate wearing safety goggles that are impractical for the clinician and uncomfortable for the patient. The electronics sub-system amounts for circa 25% of the total cost. Dedicated electronics (referred to as "DAQ" for "Data Acquisition device") with high throughput acquisition hardware are required to integrate the ultrasound and laser sub-components: triggering and transmission of the excitation signals, reception of the generated OA and reflected ultrasonic (herein also referred to as "US") acoustic pressure waves. Additionally, a high-performance graphic card is needed to reconstruct high quality OA images in real-time. The piezoelectric (i.e., ceramics, crystals, polymers and piezocomposite) ultrasound transducers sub-component amounts for circa 15% of the total cost.

Multidimensional positioning support, preferably including six degrees of freedom (6DoF), for optoacoustic medical imaging is a desired feature for volumetric (3D) imaging. Currently, add-on devices for 6DoF positioning of ultrasound handheld transducers are known which, however, are based on complex and expensive gyro technology. Further, 2D stitching technologies are known which are based on gluing tattoos to the skin: they absorb at 650 nm (below typical imaging wavelengths) so that this information can be used to generate 3D images.

Currently, the classification of OAI devices according to the prior art as laser class 3B/4 devices is cumbersome for any routine clinical application beyond radiology research-driven hospitals, in particular because a laser safety room is needed, as is a laser safety officer. Additionally, the doctor and the patient have to wear laser protecting goggles during the examination.

It is an object of present disclosure to provide an improved handheld device (also referred to as "probe") and system for optoacoustic and/or ultrasound sensing, in particular imaging, wherein information regarding a position and/or movement of the handheld device relative to an object, in particular a patient, can be detected and/or tracked in a compact, reliable and simple manner. According to an alternative or preferred object of present disclosure, the handheld device and system fulfills or meets laser safety requirements and/or standards, in particular without necessitating wearing safety goggles.

The object is achieved by a handheld device and system for optoacoustic and/or ultrasound sensing, in particular imaging, according to the independent claims. Preferred and/or alternative aspects or embodiments are subject of the dependent claims and/or the following description.

According to a first aspect, a handheld device for optoacoustic sensing, in particular imaging, of properties of an object, in particular a biological object, comprises a housing configured to be grasped by an operator's hand to be positioned and/or moved relative to the object and at least one electronic controller, wherein the following components are integrated in and/or provided on the housing: an illumination unit comprising at least one radiation source configured to illuminate the object with pulses of electromagnetic radiation; and a detection unit configured to detect ultrasonic waves emanating from the object in response to illuminating the object with the pulses of electromagnetic radiation and to convert the detected ultrasonic waves into detection signals. The handheld device further comprises at least one VCSEL sensor unit comprising a vertical-cavity surface-emitting laser (VCSEL), a laser resonator and a photodiode provided in and/or at the laser resonator and being arranged and/or configured to: illuminate the object with first electromagnetic radiation emerging from the laser resonator; allow second electromagnetic radiation, which has been reflected and/or scattered by the object in response to illuminating the object with the first electromagnetic radiation, to enter the laser resonator and mix and/or interfere with the first electromagnetic radiation, whereby an interference signal is obtained; and detect the interference signal with the photodiode. The at least one electronic controller is configured to derive, based on the detected interference signal, positional information regarding a position and/or distance and/or movement of the at least one VCSEL sensor unit and/or the handheld device relative to the object. Alternatively or additionally, the handheld device comprises at least one vertical-cavity surface-emitting laser (VCSEL) configured to illuminate the object with first electromagnetic radiation, a detector, in particular an optical or ultrasound detector, configured to, in particular incoherently, detect a response signal, in particular an optical signal or an ultrasound signal, emanating from the object in response to illuminating the object with the first electromagnetic radiation, wherein the at least one electronic controller is configured to derive, based on the detected response signal, a time measure characterizing a duration (time of flight) it takes for the first electromagnetic radiation to travel from the VCSEL to the object and, after reflection or conversion into an ultrasound wave by the object, back to the detector, and to derive positional information regarding a height position (z) and/or distance (d) of the at least one VCSEL and/or handheld device relative to the object.

According to a second aspect, a handheld device for ultrasound imaging of an object comprises a housing configured to be grasped by an operator's hand to be positioned and/or moved relative to the object, at least one electronic controller, and an ultrasound sensor unit which is integrated in and/or provided on the housing and configured to transmit ultrasound waves towards the object and to detect ultrasonic waves emanating from, in particular being reflected and/or scattered by, the object, and to convert the detected ultrasonic waves into detection signals. The handheld device further comprises at least one VCSEL sensor unit comprising a vertical-cavity surface-emitting laser (VCSEL), a laser resonator and a photodiode provided in and/or at the laser resonator and being arranged and/or configured to: illuminate the object with first electromagnetic radiation emerging from the laser resonator; allow second electromagnetic radiation, which has been reflected and/or scattered by the object in response to illuminating the object with the first electromagnetic radiation, to enter the laser resonator and mix and/or interfere with the first electromagnetic radiation, whereby an interference signal is obtained; and detect the interference signal with the photodiode. The at least one electronic controller is configured to derive, based on the detected interference signal, positional information regarding a position and/or distance and/or movement of the at least one VCSEL sensor unit and/or the handheld device relative to the object. Alternatively or additionally, the handheld device further comprises at least one vertical-cavity surface-emitting laser (VCSEL) configured to illuminate the object with first electromagnetic radiation, a detector, in particular an optical or ultrasound detector, configured to, in particular incoherently, detect a response signal, in particular an optical signal or an ultrasound signal, emanating from the object in response to illuminating the object with the first electromagnetic radiation, wherein the at least one electronic controller is configured to derive, based on the detected response signal, a time measure characterizing a duration (time of flight) it takes for the first electromagnetic radiation to travel from the VCSEL to the object and, after reflection or conversion into an ultrasound wave by the object, back to the detector, and to derive positional information regarding a height position (z) and/or distance of the at least one VCSEL and/or handheld device relative to the object.

According to a third aspect, a system for optoacoustic and/or ultrasound sensing, in particular imaging, of an object, in particular a biological object, comprises: a handheld device according to the first and/or second aspect; and a computer system, in particular a mobile computer device and/or a computer workstation and/or a cloud computing system, located outside the handheld device, in particular outside the housing of the handheld device, and being in a data communication with the at least one electronic controller, the computer system being configured to process the detection signals and/or to reconstruct an image based on the detection signals and/or to further process an image which has been reconstructed by the at least one electronic controller.

Aspects of present disclosure are preferably based on the approach of providing a handheld device for optoacoustic sensing or imaging which is not only configured to illuminate the object with electromagnetic radiation, e.g. by means of a plurality of light-emitting devices such as an array of VCSELs, to excite optoacoustic waves in the object in response to the illumination, but also to coherently detect electromagnetic radiation, which has been reflected and/or scattered by the object, by means of at least one VCSEL sensor unit (comprising a VCSEL with integrated resonator and photodiode) in such a way that positional information regarding a position and/or distance and/or movement of the at least one VCSEL sensor unit and/or the handheld device relative to the object can be derived from the coherently detected electromagnetic radiation. Alternatively or additionally, an optical or ultrasound response signal emanating from the object in response to illuminating the object with first electromagnetic radiation emitted by a vertical-cavity surface-emitting laser (VCSEL) is incoherently detected by a detector, such as a photodiode or an ultrasound detector, which is provided separately from the VCSEL, i.e. the detector (e.g. the photodiode) is not necessarily integrated in the VCSEL. Based on the incoherently detected response signal(s), a time of flight (ToF) is the derived, which characterizes the time it takes for the first electromagnetic radiation to travel from the VCSEL to the object and, after reflection or conversion into an ultrasound wave, back to the detector. A positional information regarding a height position (z) and/or distance of the at least one VCSEL and/or handheld device relative to the object is then derived from the time of flight.

Further aspects of present disclosure are preferably based on the approach of providing a handheld device for ultrasound imaging including such a VCSEL sensor unit (VCSEL with integrated resonator and photodiode) for coherently detecting the reflected electromagnetic radiation and/or a VCSEL and a (separate) detector for incoherently detecting the optical or ultrasound response signal(s) in response to illuminating the object with electromagnetic radiation, wherein the above and following explanations regarding the device for optoacoustic sensing or imaging apply mutatis mutandis to the handheld device for ultrasound imaging.

Preferably, the VCSEL sensor unit comprises at least one VCSEL, which is preferably designed as a single-mode VCSEL, comprising an optical resonator and an integrated photodiode to enable a so-called self-mixing interference (SMI), also referred to as self-mixing technology, wherein the optical resonator of the VCSEL sensor unit captures a reflection and/or scattering of the laser beam at the object and mixes it with the light in the resonator. The VCSEL sensor unit detects and/or measures the variation in power caused by the interference, and a processor calculates the speed of movement (between the probe/VCSEL sensor unit and the object) from the difference in frequency. The direction of movement of the object relative to the handheld probe can be deduced from the modulation of the wavelength due to the Doppler effect on the backscattered light. In this way, the VCSEL sensor unit can be used for a high-precision tracking of the handheld probe relative to the object and vice versa, similarly to high-end tracking of a computer mouse using the Doppler effect.

Alternatively or additionally, the distance, in particular the absolute distance, between the handheld device and the object can be monitored by time-of-flight (ToF) measurement using the integrated photodiode and/or a separate photodiode or ultrasound wave detector, which also implies and/or provides information regarding a contact of the device with the skin, which can optionally be used if, e.g., a class 1c laser classification of the device shall be enabled. In other words, distance information relating to a distance between the handheld probe and the object can be used as an indicator that the handheld device is in contact with and/or close to the object (e.g. when the distance is zero or close to zero) or not (e.g. when the distance is not zero). In this way, optionally together with a second contact sensor, a laser class 1c shut-off can be ensured.

Preferably, the at least one VCSEL sensor unit can be controlled to monitor a lateral or side motion of the handheld probe relative to the object during which a plurality of slice and/or partial optoacoustic 2D images of the object are obtained; the motion and/or position information obtained in this way can then be used for stitching the respectively obtained, e.g., 2D optoacoustic images together to obtain, e.g., a 3D image. Preferably, an artificial intelligence (Al) supported algorithm can be used to find the best position in the tissue based on the 3D image for the final measurement and guiding the user to the optimum position for quantification purposes.

In other words, aspects of present disclosure are preferably based on the approach of using a specifically designed VCSEL, which is also referred to as "VCSEL sensor unit", as a positioning sensor, wherein a coherent superposition of resonator mode with Doppler-shifted backscattered light can be used to monitor the positioning in ultrasound and optoacoustic imaging. Advantageously, a 3D image can be generated by using compounding, extrapolation, and/or stitching of 2D images for which positional information has been acquired in this way. Further, out of the 3D image, a computerized method (e.g., a neural network) can identify the best position in the tissue for a specific application (e.g. determining blood oxygenation or glucose concentration), wherein the system software guides the user automatically to the optimal position. As an alternative or in addition to a VCSEL sensor unit for coherent detection, by means of a VCSEL and a detector for incoherent detection (e.g. a separate photodiode or ultrasound sensor), an axial distance to the skin of a patient and/or an absolute axial distance to the skin of a patient can be obtained by means of a time-of-flight (ToF) measurement. In this way, the VCSEL and detector for incoherent detection can be used and/or regarded as one of preferably two contact sensors for enabling a laser class 1c classification of the device.

Preferably, one to four single-mode VCSEL sensor units are positioned at, preferably the corners of, the handheld (ultrasound and/or optoacoustic) sensing or imaging device. Preferably, the integrated electronics of the VCSEL sensor units' driver(s) generate the positioning information of all one to four VCSEL sensor units. The electronic controller, preferably a FPGA in the data acquisition unit, preferably calculates the position and provides this positioning information to each individual 2D slice raw data set. The imaging software preferably generates 3D images and/or a neural network may be provided to identify the best position for measuring/quantifying for a specific application.

Preferably, the axial information from the VCSEL sensor unit and/or photodiode electronics and/or ToF measurements may be permanently monitored together with another contact and/or distance sensor: if one of the two distance and/or contact sensors shows a noncontact situation, the laser power is switched of via two independently hard-wired connections as a safety mechanism.

In summary, present disclosure provides an improved handheld device and system for optoacoustic and/or ultrasound sensing, in particular imaging, wherein information regarding a position and/or movement of the handheld device relative to an object, in particular a patient, can be detected and/or tracked in a compact, reliable and simple manner and can optionally also be used for enabling a specific laser classification, in particular class 1c, of the device.

In some instances of present disclosure, in particular in the context of determining positional information and/or motion tracking, the term "VCSEL sensor unit" (which does not only include a VCSEL but also a resonator and a photodiode) is abbreviated as "VCSEL" for the sake of simplification.

Preferably, the at least one electronic controller is configured to control the at least one radiation source to emit the pulses of electromagnetic radiation dependent on the derived positional information. In this way, the emission of the pulsed electromagnetic radiation, in particular the laser radiation, can be specifically, e.g. depending on the application or purpose or required property of the device, controlled by considering the derived positional information.

Preferably, the at least one controller may comprise an axial controller for controlling the device and/or image reconstruction dependent on axial (z) or distance information, and a lateral which is independent from the axial controller and configured to control the device and/or image reconstruction dependent on lateral (x, y) information.

Preferably, the at least one electronic controller is configured to control the at least one radiation source i) to emit the pulses of electromagnetic radiation only if the derived positional information, in particular axial (z) or distance information, is indicative that the handheld device, in particular the housing, is in contact with and/or close to the object, and/or ii) to stop emitting the pulses of electromagnetic radiation if the derived positional information, in particular axial (z) or distance information, is indicative that the handheld device, in particular the housing, is not in contact with and/or close to the object. By this means, a shut-off of the emission of laser radiation is realized in a particularly reliable manner to enable a class 1c laser classification of the device.

Preferably, a contact sensor is integrated in and/or provided on the housing and configured to detect contact information which is indicative whether the handheld device, in particular the housing, is in contact with the object or not. For example, the contact sensor can be a mechanical switch or optical sensor such as a light barrier. Preferably, the sensor is a conductivity or capacity sensor which is configured to monitor an electrical conductivity or capacity, respectively, which depends on whether the sensor is in contact with the object or not, or whether the sensor is being removed from the object or placed on the object causing a change of conductivity or capacity, respectively. Preferably, the at least one electronic controller is configured to control the at least one radiation source i) to emit the pulses of electromagnetic radiation only if both the derived positional information, in particular axial (z) or distance information, and the contact information are indicative that the handheld device, in particular the housing, is in contact with or close to the object, respectively, and/or ii) to stop emitting the pulses of electromagnetic radiation if either the derived positional information, in particular axial (z) or distance information, or the contact information is indicative that the handheld device, in particular the housing, is not in contact with or not close to the object, respectively. By this means, a shut-off of the emission of laser radiation is realized in a particularly reliable manner to enable a class 1c laser classification of the handheld device.

Alternatively or additionally to deriving positional information, in particular position(s) and/or motion of the device along the x-y plane, based on coherent detection using the Doppler shift information measured by the VCSEL sensor unit, an axial (z) or distance information, in particular an absolute distance value, is obtained by means of a ToF measurement, i.e. by measuring the elapsed time between the emission of a pulse (generated by a VCSEL) towards the object and incoherent detection of reflected electromagnetic radiation in response thereto by an optical detector, such as a photodiode.

Preferably, a ToF measurement can be realized by providing an optical detector, in particular a photodiode (which is preferably different from the photodiode integrated in the VCSEL sensor unit), which is arranged and/or configured to detect electromagnetic radiation which has been reflected by the object in response to illuminating the object with pulses of electromagnetic radiation emitted by the at least one VCSEL(which may be a VCSEL of the radiation source or a VCSEL in addition to and/or separately from the radiation source), wherein the pulses of electromagnetic radiation have a pulse repetition rate between 100 Hz and 10 kHz, in particular between 500 Hz and 2 kHz, preferably approximately 1 kHz. Preferably, the at least one electronic controller is configured to receive, from the optical detector, signals corresponding to the detected electromagnetic radiation which has been reflected by the object, to derive, based on the received signals, a distance (z), in particular an absolute distance, of the handheld device relative to the object, and to control the at least one VCSEL of the radiation source to continue emitting the pulses of electromagnetic radiation only if the derived distance (z) is indicative that the handheld device, in particular the housing, is in contact with and/or close to the object, and/or to stop emitting the pulses of electromagnetic radiation if the derived distance (z) is indicative that the handheld device, in particular the housing, is not in contact with and/or not close to the object.

Using VCSELs emitting pulses with a relatively high pulse repetition rate, e.g. 1 kHz, and with relatively low energy allows for switching off the VCSELs within a very short time required for a single shot (pulse), so that a damage of the eye can be reliably prevented. In distinction to this, e.g., a YAG-OPO emitting pulses with relatively low repetition rate of 25 Hz and higher energy would require a considerably longer time for a single shot and, therefore, for switching off the laser emission, so that a single shot can damage the eye.

Preferably, the at least one controller can be configured to not only consider the derived distance (z) but also contact information provided by a contact sensor as described above when controlling the at least one VCSEL of the radiation source. In these preferred embodiments, laser class 1c can be achieved without necessarily relying on axial (z) or distance information derived from the interference signal of the VCSEL sensor unit, because the at least one VCSEL of the radiation source together with the additional optical sensor provide the relevant ToF information. On the other hand, it is possible to rely on both the axial (z) or distance information derived from the interference signal of the VCSEL sensor unit and the axial (z) or distance information obtained by ToF measurement.

Preferably, the at least one electronic controller is configured to derive the positional information for each of a plurality of different lateral (x, y) positions of the handheld device while being moved laterally relative to the object, and/or each of a plurality of different depth (z) positions of the handheld device while being moved relative, in particular towards and/or away from the object, and/or each of a plurality of different angular positions and/or directions relative to the object while being moved relative to the object. In this way, positional information up to six degrees of freedom (6DoF) can be determined.

Preferably, the at least one electronic controller is configured to reconstruct a plurality of first optoacoustic images, in particular two-dimensional optoacoustic images, based on the detection signals obtained for each of the plurality of different lateral positions, and/or reconstruct a second optoacoustic image, in particular a three-dimensional optoacoustic image, by stitching the first optoacoustic images using the positional information derived for each of the different lateral positions. In other words, optoacoustic images, e.g. 2D images, which were reconstructed from detection signals corresponding to optoacoustic waves which detected in response to illuminating the object with the pulsed electromagnetic radiation, are stitched together to a compound optoacoustic image, e.g. a 3D image, based on the respective positional information which has been derived from interference signal(s) which was or were detected during acquisition of the detection signals when the VCSEL and/or handheld device was in the respective position(s) relative to the object.

Preferably, the at least one radiation source is and/or comprises at least one vertical-cavity surface-emitting laser (VCSEL) and/or the detection unit comprises at least one micromachined ultrasonic transducer, in particular at least one capacitive micromachined ultrasonic transducer (CMUT) or piezoelectric micromachined ultrasonic transducer (PMUT).

Preferably, the position and orientation of the transducer and/or handheld device relative to the surface of the imaged object is continuously monitored during scanning (i.e. OA or US imaging) by means of motion tracking. This will be illustrated by a figure and described further below. Preferably, the derived positional information provides at least one of four distinct types of positional information, each serving a specific purpose, as follows:
1. Contact information (z). Detects or contains information whether the transducer membrane and/or handheld device is in contact with the patient skin. The purpose is, e.g., to trigger an alert and/or to stop emitting laser radiation (i.e. the pulsed electromagnetic radiation) when contact is not maintained.
2. Positional information. Information about the position and orientation of the transducer surface and/or handheld device with respect to the patient skin. The purpose is to map each pixel of the two-dimensional image field of view of any acquired frame into the three-dimensional space. Preferably, six degrees of freedom are used (translations across and rotations around the three dimensions of space).
3. Immobility information. Indicates or contains information whether the transducer and/or handheld device is kept static through time with respect to the patient skin. The purpose is to guide applications that require a temporal acquisition of a fixed region of interest (such as for instance, acquiring a collection of several co-registered wavelength frames for spectral unmixing, and/or acquiring a collection of coregistered scans to average the signals and increase the signal-to-noise ratio).
4. Reproducibility information. Confirms or contains information that a second set of measurements, which is potentially conducted minutes or days apart a previous measurement, is taken at the same location (position and orientation), for instance to observe the same site before and after a clinical intervention.

Preferably, motion tracking of types 1-3 is principally based on information derived from the VCSEL sensor unit(s) (wherein axial (z) or distance information can, alternatively or additionally, be provided by ToF measurement(s) as described above) and require a constant contact of the probe onto the patient skin. Preferably, motion tracking of type 4 is based on information derived from optoacoustic and/or ultrasound image features and can support the probe being removed from the skin surface and replaced again.

Preferably, the principal source of information to derive motion tracking (for types 1-3) are the probe's VCSEL sensor units and/or VCSEL and incoherent detector (wherein ToF measurement can only provide z). In complement to the VCSEL sensor units and/or VCSEL and incoherent detector, one can also rely (especially for type 4) on a combination of the following sources of information to achieve motion tracking: image-based information either from reconstructed optoacoustic and/or ultrasound data; signal-based information either from acquired optoacoustic and/or ultrasound sinograms; gyroscope; accelerometer. In contrast to the prior art, the handheld device and system according to the present disclosure advantageously eliminates the need for the following sources of information to achieve motion tracking: electromagnetic tracking; optical tracking; mechanical positional guidance; fiducial landmarks such as tattoos.

Preferably, motion tracking is used to derive the position and orientation of the imaged plane from the spatial positioning of the transducer and/or handheld device, and to incorporate this information as part of the metadata of the recorded optoacoustic and/or ultrasound signals. Therefore, it becomes possible to relate the spatial coordinate between pixels of different frames acquired during the same sweep.

### Optoacoustic quantification with a local vs. global perspective

Quantification of optoacoustic images is generally conducted locally. Considering jointly several regions (with different transducer poses and orientations) of the same anatomy is a valid way to improve quantification performance and clinical applicability. For instance, averaging muscle tissue oxygenation over adjacent positions would reduce variability in assessment of muscle ischemia and also enable understanding of the spatial variability of ischemia. This would enable better discrimination of healthy versus diseased individuals, or allow better discrimination of the extent of disease, in a variety of diseases that present with muscle ischemia, including but not limited to peripheral artery disease, neuromuscular disease, fibromyalgia, sarcopenia, compartment syndrome, sickle cell anemia, rhabdomyolysis, cerebral palsy, sepsis and myositis.

### Imaging pipeline

1. image reconstruction: Preferably, optoacoustic and/or ultrasound images are reconstructed in real-time with either a deep-learning-based algorithm or an analytic algorithm capable of achieving state-of-the-art quality at a frame-rate greater than 5 Hz.
2. Object recognition: Preferably, each individual frame is automatically analyzed to determine i) whether the current field of view contains the target object, and ii) whether the current sweep of images covers a sufficiently broad volume to sample the target object.
3. Segmentation of the region of interest (ROI): Preferably, for each image, the region of interest that contains the target object is automatically segmented to exclude the background.
4. Confidence evaluation: Preferably, for each image, the segmented ROI is automatically analyzed (in terms of quantification of e.g., signal-to-noise ratio, light fluence effect, and potential presence of detrimental artifacts) to determine whether data can be reliably used or shall be discarded instead.
5. Volume compounding: Preferably, the spatial information derived from motion tracking can be used to automatically compound the collection of acquired 2D images into a 3D volume representation by means of image concatenation, stitching and/or inpainting.
6. image quantification: Preferably, hyperspectral unmixing algorithms are applied to the acquired scans to quantify intrinsic chromophore concentration. For improved robustness and usability, the quantified values are interpreted by collectively considering the ensemble of segmented ROIs of all images acquired during the sweep.

Preferably, all steps of the imaging pipeline can be computed in an automated fashion and at a frame rate greater than 5 Hz.

Preferably, in a laser class 1c handheld device, two independent sensors in the handheld switch off the laser, wherein one of the sensors are one or more standalone VCSELs with an integrated photodiode or a VCSEL array with one or more integrated photodiodes in a VCSEL mesa for incoherent or coherent detection of the backscattered light (also referred to as "VCSEL sensor unit(s)").

Preferably, for the purpose of 3D stitching of 2D images, one or more standalone VCSEL(s) with an integrated photodiode or a VCSEL array with one or more integrated photodiodes in a VCSEL mesa (also referred to as "VCSEL sensor unit(s)") are used for coherent detection of the backscattered light to measure the motion of the handheld and to guide the measurement position of the user. Preferably, AI guidance is provided to find the optimum position in the 3D images for quantification of oxygenation in tissue. Reconstruction algorithms such as efficient DeepMB can be used to allow for high frame rate > 5 Hz.

Preferably, for quantification of oxygenation in tissue, reconstruction algorithms, such as *Efficient Deep Model-Based Optoacoustic Image Reconstruction* as described by C. Dehner and G. Zahnd in arXiv:2408.07109 [eess.IV] (https://doi.org/10.48550/arXiv.2408.07109), can be used to allow for high frame rate > 5 Hz.

Preferably, the detection unit can also be configured to become, in alternance, an active excitation unit to transmit ultrasonic pulses into the object.

Preferably, the illumination unit additionally comprises a plurality of optical fibers configured to deliver light from a light source external to the housing of the handheld device.

Preferably, a first radiation source remains active during use to provide at least one interference signal (containing positional information), and a second radiation source is modulated or turned off by the controller in response to the position and/or distance and/or movement (determined based on the positional information).

Preferably, the controller uses the signals produced by the detection unit and/or VCSEL sensor unit to determine if the membrane is in contact with the object.

Preferably, the controller uses the signals produced by the detection unit and/or VCSEL sensor unit to determine if the housing stays immobile with respect to the object, by monitoring the absence of change of the housing's position and orientation along time.

Preferably, the controller comprises and/or receives signals from a sensor for detecting the distance and/or contact pressure between the object and the membrane.

Preferably, the controller comprises and/or receives signals from a sensor for detecting electrical conductivity of the object or changes in conductivity when the system is removed from the object.

Preferably, a plurality of VCSEL sensor units for coherent detection and/or VCSELs with incoherent detector(s) (e.g. VCSEL/photodiode pairs) are provided which produce independent interference or response signals, respectively.

Preferably, the at least one controller additionally uses information from recorded optoacoustic or ultrasound signals to estimate the position and/or distance and/or movement.

Preferably, the at least one controller is configured to continuously derive the position and orientation of the imaged plane from the spatial positioning of the transducer, and to incorporate this information as part of the metadata of the recorded optoacoustic and/or ultrasound signals.

Preferably, the at least one controller is configured to provide an information to the user about the distance and/or position and/or movement of the housing relative to the object.

Preferably, the information comprises an optimal location for estimating/imaging a parameter of the object, completeness of a 3D reconstruction, etc.

Preferably, in the information the parameter of the object is oxygenation of hemoglobin, a ratio of different photoabsorbers, a concentration of a photoabsorber, etc.

Preferably, the at least one controller is configured to reconstruct optoacoustic images from the recorded signals at a frame rate above 5 Hz.

Preferably, the at least one controller is configured to reconstruct ultrasound images from the recorded signals at a frame rate above 5 Hz.

Preferably, the at least one controller is configured to additionally use information from reconstructed optoacoustic or ultrasound images to estimate the position and/or distance and/or movement.

Preferably, the at least one electronic controller is configured to derive the positional information for each of a plurality of different positions of the handheld device while being moved relative to the object, and to reconstruct a plurality of first optoacoustic and/or ultrasound images, in particular two-dimensional images, based on the detection signals obtained for each of the plurality of different positions and/or to reconstruct a three-dimensional optoacoustic and/or ultrasound image, by compounding (namely, concatenating, stitching, and/or inpainting) a plurality of reconstructed optoacoustic and/or ultrasound images using the positional information derived for each of the different positions.

Preferably, the at least one controller provides information to the user based on the quality, usability, or reliability of the reconstructed two-dimensional optoacoustic and/or ultrasound images, and/or of the compounded three-dimensional optoacoustic and/or ultrasound volumes.

Optionally, a skin type detection/compensation can be performed: The VCSELs sensor unit(s) and/or photodiode(s) could additionally detect the amplitude of backscattered light. This could help to determine the general lightness/darkness (albedo) of an object. In particular, this could be used to assess the skin pigmentation level of a patient (e.g., predict a Fitzpatrick skin phototype, ITA degree, or other skin classification system). The albedo or skin classification could be used to make corrections in the image or as input to a diagnostic score.

Further additional or alternative aspects, advantages, features and examples of the present disclosure will be apparent from the following description of following figures, wherein:
- Fig. 1: shows an example of a handheld device 1 for optoacoustic imaging;
- Fig. 2: shows a schematic representation of perspective views and (magnified) cross-sectional views of an example of a coherent VCSEL sensor unit;
- Fig. 3: shows definitions/requirements of laser class 1c according to IEC 60825-1;
- Fig. 4: shows a schematic representation of a six-degrees-of-freedom (6DoF) tracking of a handheld device using VCSEL sensor unit(s); and
- Fig. 5: shows an example of a handheld device for ultrasound imaging.

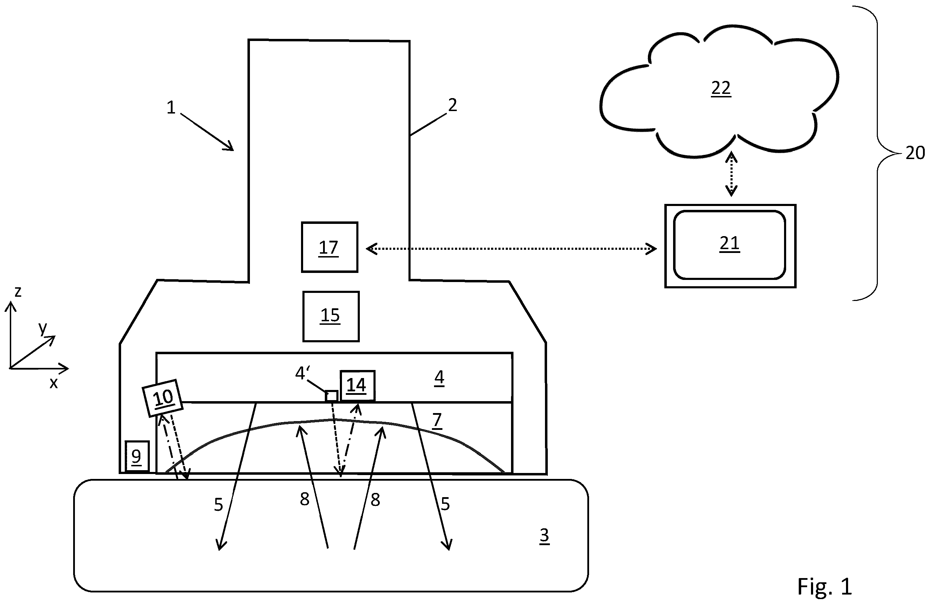

Figure 1 shows an example of a handheld device 1 for optoacoustic imaging of an object in a schematic cross-sectional view. The device 1 comprises a housing 2 having a form and a size so that it can be grasped by an operator's hand (not shown) to be positioned and/or moved relative to an object 3, for example a patient, to be investigated.

The housing 2 contains an illumination unit 4 comprising at least one radiation source configured to illuminate the object 3 with time-varying, in particular pulsed and/or a modulated, electromagnetic radiation 5, and a detection unit 7 configured to detect ultrasonic waves 8 emanating from the object 3 in response to illuminating the object 3 with the time-varying electromagnetic radiation 5 and to convert the detected ultrasonic waves 8 into responding detection signals. Preferably, the detection unit 7 comprises a, preferably concave, array of piezoelectric or micromachined ultrasonic transducers, in particular capacitive micromachined ultrasonic transducers (CMUTs) or piezoelectric micromachined ultrasonic transducers (PMUTs) or regular piezoelectric elements. Preferably, the radiation source comprises an array of vertical-cavity surface-emitting lasers (VCSELs) or a plurality of optical fibers configured to guide electromagnetic radiation emitted by an external laser source (not shown), e.g. a YAG-OPO provided in a cart, into the handheld device 1 for illuminating the object 3.

The handheld device 1 further comprises one or more VCSEL sensor units 10 each of which being configured both to irradiate the object 3 with electromagnetic radiation (dashed arrows) emitted by a VCSEL of the VCSEL sensor unit 10 and to detect electromagnetic radiation (dashed-dotted arrows) which has been reflected by the object 3 in response to irradiating the object 3. Preferably, the VCSEL sensor units 10 are configured to coherently detect the backscattered radiation and provide an according interference signal resulting from a superposition (constructive or destructive interference) of the emitted radiation and the backscattered radiation.

Alternatively or additionally to providing VCSEL sensor units 10, the handheld device 1 may be configured to determine a, preferably absolute, distance between the handheld device 1 and the object 3 by means of a time-of-flight (ToF) measurement, wherein backscattered radiation or optoacoustic waves emanating from the object 3 is/are incoherently detected to determine a time ("time of flight") it takes for electromagnetic radiation to travel from a VCSEL to the object 3 and, after reflection or conversion into optoacoustic waves by the object 3, back to a incoherent detector, such as an separate photodiode or an ultrasound detector. The determined time of flight can then be used to derive the, preferably absolute, distance between the handheld device 1 and the object 3. Preferably, the radiation-emitting VCSEL may be one of the VCSELs of the illumination unit 4 or be a separate VCSEL dedicated for ToF measurement. Preferably, the incoherent detector may be an optical detector, such as a photodiode, or an ultrasound detector which can be given by one or more ultrasonic transducers of the detection unit 7 or by a separate ultrasound detector. In the example shown in figure 1, a VCSEL 4' (enlarged for illustrative purposes) which is a part of the radiation source of the illumination unit 4 irradiates the object 3 with electromagnetic radiation (dotted arrow), wherein the backscattered radiation (dashed-dotted arrows) is incoherently detected by a separate photodiode 14 to provide a time-of-flight information based on which the distance (along the z dimension) between the handheld device 1 and the object 3 and be derived. In an alternative embodiment (not shown), rather than using a VCSEL 4' of the illumination unit 4, the component 14 may comprise a separate VCSEL (dedicated for ToF measurement) for illuminating the object 3 in addition to a photodiode for incoherent detection of the backscattered radiation.

In the example shown in figure 1, two VCSEL sensor units 10 are provided at different positions on the illumination unit 4, e.g. on a front surface of a VCSEL array module, wherein the VCSEL sensor units 10 are spaced apart and inclined with respect to the front face (e.g. a membrane) of the housing 2 such that the emitted electromagnetic radiation impinges at angles different from 90° onto the surface of the object 3, e.g. the skin of a patient. In this way, it can be reliably ensured to detect a motion of the handheld device 1 parallel to the surface of the object 3. Preferably, the two sensor units 10 are angled in ideally perpendicular planes, i.e. the electromagnetic radiation emitted from each of the two outer sensor units 10 impinges at an angle of preferably 45° onto the surface of the object 3. Since the two sensor units 10 are spaced some distance apart, they provide positional information based on which a twisting of the handheld device 1 around the axis normal to the skin surface (rotation around the z axis) can be recognized.

Further, it is possible to provide and arrange three VCSEL sensor units 10 such that the planes where the VCSEL sensor units 10 are angled in do not meet at the same line. A simple implementation would be for three angled sensors to detect motion along three lines along the skin that form a triangle.

Further, depending on the application and/or kind of positional information that is desired, it may be preferred to provide only one VCSEL sensor unit 10, or to provide three or four VCSEL sensor units 10, in particular if some redundancy in the acquired positional information is preferred.

Further, it may be preferred to arrange the VCSEL sensor units 10 at positions different to the positions exemplarily shown in figure 1. For example, the VCSEL sensor units 10 may be arranged close to the front face (membrane) of the housing 2 and/or mounted on the outside of the housing 2.

Preferably, the handheld device 1 further comprises at least one electronic controller 15, which is preferably integrated in the housing 2 and configured to derive, based on the interference signal(s) and/or ToF measurement(s) of the VCSEL sensor units 10, a positional information regarding a position and/or distance and/or movement of the handheld device 1 relative to the object 3.

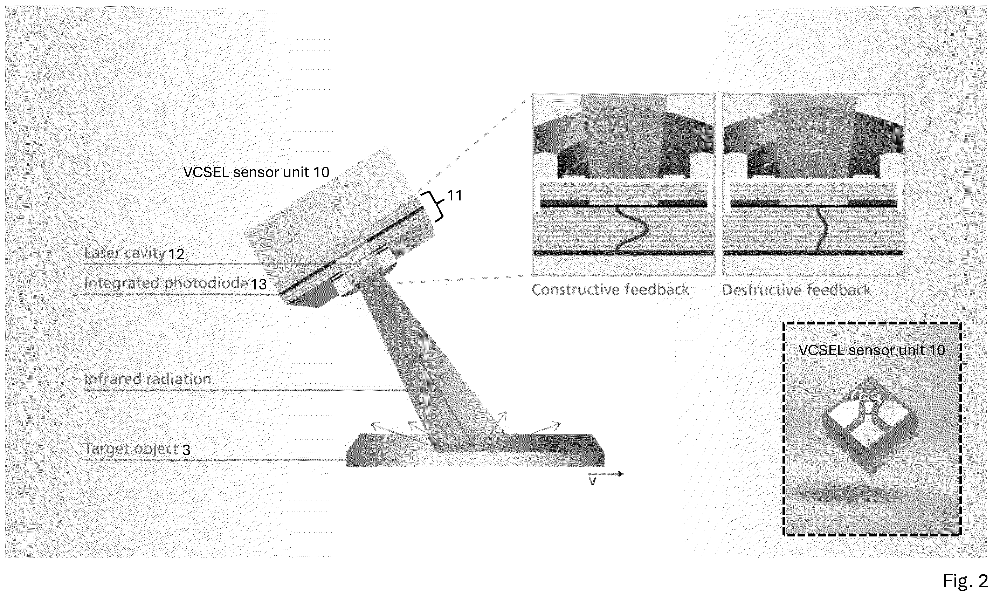

Figure 2 shows a schematic representation of perspective views and (magnified) cross-sectional views of an example of a VCSEL sensor unit 10 which provides interference signals containing positional information, e.g. regarding coordinates of a position (x, y, z) and/or time-of-flight information (z direction, distance d) and/or an orientation (α, β, χ) and/or a distance of the handheld device relative to a target object 3 and/or a relative movement and/or acceleration and/or deceleration.

The coherent VCSEL sensor unit 10 comprises a vertical-cavity surface-emitting laser (VCSEL) 11, a laser cavity 12, also referred to as "laser resonator" herein, and an integrated photodiode 13 provided in and/or at the laser resonator 12. The VCSEL 11 is configured to generate electromagnetic radiation (e.g. infrared radiation), which is also referred to as "first electromagnetic radiation" herein and, after passing the laser resonator 12, illuminates the target object 3, for example the skin of a patient. A part of the electromagnetic radiation, which is scattered and/or reflected by the object 3 and also referred to as "second electromagnetic radiation" herein, re-enters the laser resonator 12 of the VCSEL sensor unit 10 and mixes and/or interferes with the first electromagnetic radiation, whereby an interference signal is obtained, which is detected by the photodiode 13.

As apparent from the cross-sectional views of the VCSEL sensor unit (see the two upper right insets of figure 2), the interference signal is indicative of a constructive or destructive feedback (or interference) which depends on the distance, including changes of the distance, between the VCSEL sensor unit 10 and the target object 3. In this way, the VCSEL sensor unit 10 allows to coherently measure a Doppler shift which is indicative of a relative movement between the VCSEL sensor unit 10 and the target object 3. An electronic controller (see figure 1), e.g. a computer or microcontroller, is configured to derive, based on the detected interference signal, the positional information regarding the position and/or distance and/or movement of the at least one VCSEL sensor unit 10 and/or the handheld device 1 relative to the object 3.

The lower right inset (with dashed frame) of figure 2 shows a perspective view of an example of an optional VCSEL sensor unit 10 implementation for a Doppler shift measurement of x-y movement (as described above) and z movement by means of the time-of-flight (ToF) principle (i.e. measuring the time it takes for a wave to travel from the VCSEL sensor unit (as the time-of-flight sensor) to the object and back) with two individual VCSELs which are integrated in a compact package.

As further illustrated by figure 1, the controller 15 may be configured to control the at least one radiation source, e.g. the VCSEL array, the illumination unit 4 to emit the electromagnetic radiation 5 dependent on the derived positional information, wherein the positional information preferably characterizes a position along the z direction (orthogonal to a front face, e.g. a membrane, of the handheld device 1) or regarding a distance between the handheld device 1 and the object 3. Preferably, the at least one radiation source is controlled to emit the electromagnetic radiation only if the derived z position and/or distance is indicative that the handheld device 1, in particular the housing 2, is in contact with and/or close to the object 3 or, respectively, to stop emitting the electromagnetic radiation if the derived z position and/or distance is indicative that the handheld device 1, in particular the housing 2, is not in contact with and/or not close to the object 3.

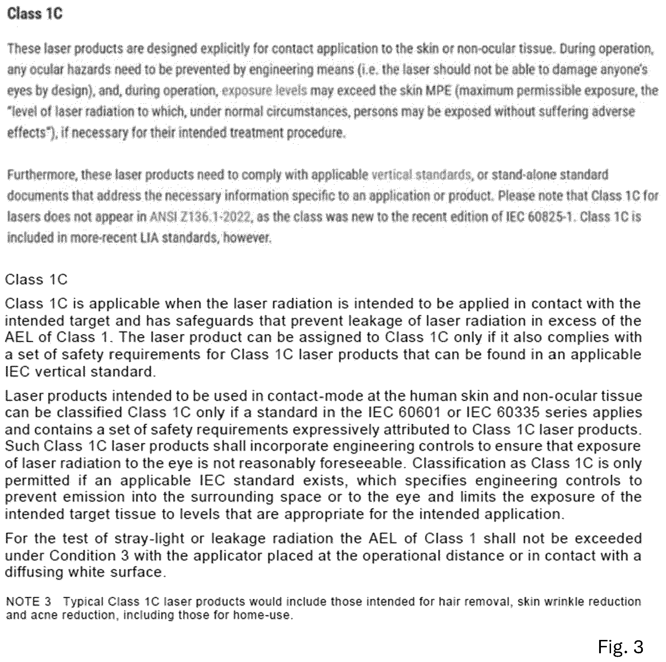

Optionally, the derived z position and/or distance can be used to achieve laser class 1c for the handheld device 1. For definitions/requirements of laser class 1c according to IEC 60825-1, see figure 3.

Preferably, two independent sensors are provided, namely a coherent VCSEL sensor unit 10 or an incoherent photodiode 14 or the ToF information from the ultrasound signal (detected by an ultrasound sensor, e.g. a transducer of the detection unit 7) and an additional distance or contact sensor 9, e.g., a conductivity sensor, for measuring a distance or contact between the handheld device 1 and the object 3.

Preferably, the radiation source, e.g. in particular the laser source provided in the cart or the VCSEL array(s) of the illumination unit 4 integrated in the handheld device 1, is controlled to emit the electromagnetic radiation 5 only if both the derived z position or distance information provided by the additional distance or contact sensor 9 are indicative that the handheld device 1, in particular its housing 2, is in contact with and/or close to the object 3 or, respectively, to stop emitting the electromagnetic radiation in all other cases. In this way, laser class 1c for the handheld device 1 can be achieved in a very reliable manner.

Preferably, the controller 15 may be configured to process the detection signals corresponding to the detected ultrasonic waves 8, e.g. by reconstructing 2D and/or 3D images based on the detection signals and the positional information. Preferably, positional information is derived for: each of a plurality of different lateral (x, y) positions of the handheld device 1 while being moved laterally relative to the object 3, and/or each of a plurality of different height (z) positions of the handheld device 1 while being moved relative, in particular towards and/or away from the object 3, and/or each of a plurality of different angular positions (α, β, γ) and/or directions relative to the object 3 while being moved relative to the object 3. Subsequently, a plurality of first optoacoustic images, in particular two-dimensional optoacoustic images, are reconstructed based on the detection signals obtained for each of the plurality of different lateral positions (x, y), and a second optoacoustic image, in particular a three-dimensional optoacoustic image, is reconstructed by stitching the first optoacoustic images using the positional information derived for each of the different lateral positions (x, y).

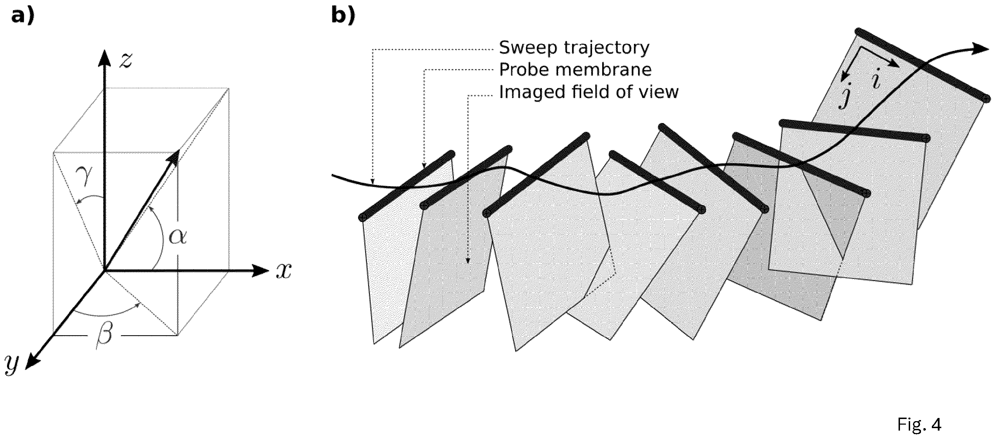

This is exemplarily illustrated in figure 4, which shows a schematic representation of a six-degrees-of-freedom (6DoF) tracking of the handheld device 1 using the VCSEL sensor unit(s). As illustrated in figure 4a, the spatial coordinates preferably indicate three translational directions (x, y, z) and three rotational directions (α, β, γ). Further, figure 4b exemplarily illustrates a sweep trajectory of the handheld device 1, or specifically the probe membrane provided at the contact surface (front face) of the handheld device 1. Preferably, each pixel of the imaged field of view at coordinates (i, j) can be mapped into the spatial coordinate system (x, y, z), and each image plane can be oriented using angles (α, β, γ). Preferably, the electronic controller (see figure 1) and/or a computer such as a PC or a microcontroller is configured to reconstruct a plurality of 2D optoacoustic images based on the detection signals obtained for each of the plurality of different positions and/or orientations (x, y, z, α, β, γ) of the handheld device 1, and to reconstruct a 3D optoacoustic image by combining and/or stitching the 2D optoacoustic images using the positional information derived for each of the different positions and/or orientations (x, y, z, α, β, γ).

Preferably, the housing 2 of the handheld device 1 (see figure 1) preferably includes an interface unit 17 which is configured to establish a wireless and/or wired data communication between the electronic controller 15 and a computer system 20, in particular a computer device 21 and/or a computer workstation and/or a cloud computing system 22, which is located outside the housing 2 of the handheld device 1 and configured to further process the signals and/or images.

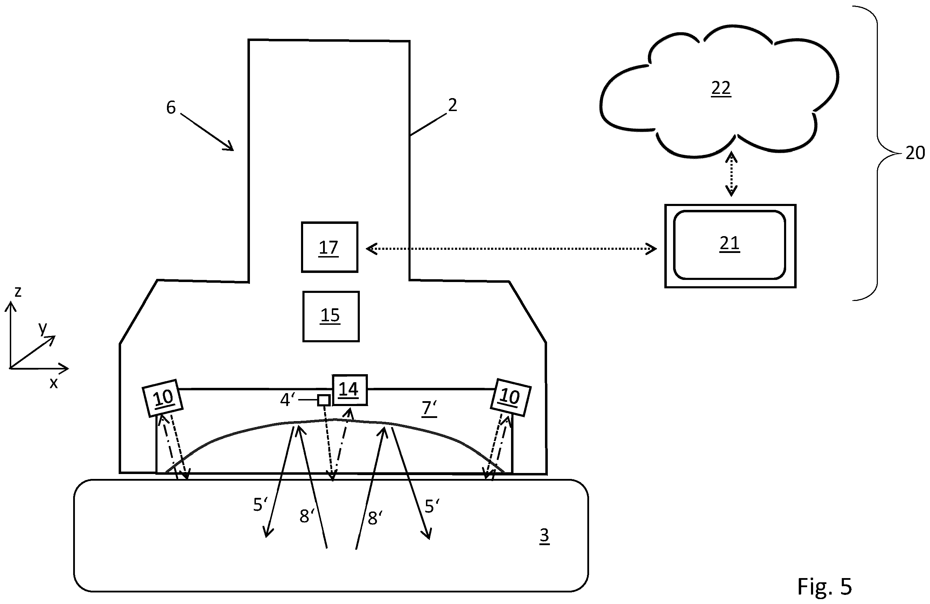

Figure 5 shows an example of a handheld device 6 for ultrasound imaging of an object in a schematic cross-sectional view. In distinction to the handheld device 1 shown in figure 1, the handheld device 6 is configured for classical ultrasound imaging, wherein instead of an illumination unit 4 and a receive-only detection unit 7 (see figure 1), a transmit-and-receive ultrasound sensor unit 7' is integrated in the housing 2 and configured to both transmit ultrasound waves 5' towards the object 3 and to detect ultrasonic waves 8' emanating from the object 3 in response thereto.

Apart from this difference, the above explanations with reference to figures 1, 2 and 4 apply accordingly to the handheld device 6. In particular, as with the handheld device 1, providing one or more VCSEL sensor units 10 and/or at least one VCSEL 4' and photodiode 14 (for deriving the distance (z) based on time-of-flight measurement) for tracking the position and/or orientation and/or movement of the handheld device 6 allows for generating three-dimensional ultrasound images from a plurality of two-dimensional ultrasound images acquired at a plurality of different positions and/or orientations.

## Claims

1. A handheld device (1) for optoacoustic imaging of an object (3), the device (1) comprising
- a housing (2) configured to be grasped by an operator's hand to be positioned and/or moved relative to the object (3), and
- at least one electronic controller (15),
wherein the following components are integrated in and/or provided on the housing (2):
- an illumination unit (4) comprising at least one radiation source configured to illuminate the object (3) with pulses of electromagnetic radiation (5), and
- a detection unit (7) configured to detect ultrasonic waves (8) emanating from the object (3) in response to illuminating the object (3) with the pulses of electromagnetic radiation (5) and to convert the detected ultrasonic waves (8) into detection signals,
**characterized by**
i) at least one VCSEL sensor unit (10) comprising a vertical-cavity surface-emitting laser (VCSEL), a laser resonator (12) and a photodiode (13) provided in and/or at the laser resonator (12) and being arranged and/or configured to: illuminate the object (3) with first electromagnetic radiation emerging from the laser resonator (12); allow second electromagnetic radiation, which has been reflected and/or scattered by the object (3) in response to illuminating the object (3) with the first electromagnetic radiation, to enter the laser resonator (12) and mix and/or interfere with the first electromagnetic radiation, whereby an interference signal is obtained; and detect the interference signal with the photodiode (13), wherein the at least one electronic controller (15) is configured to derive, based on the detected interference signal, positional information regarding a position and/or distance and/or movement of the at least one VCSEL sensor unit (10) and/or handheld device (1) relative to the object (3), and/or
ii) at least one vertical-cavity surface-emitting laser (VCSEL) (4') configured to illuminate the object (3) with first electromagnetic radiation, a detector (7; 14), in particular an optical or ultrasound detector, configured to, in particular incoherently, detect a response signal, in particular an optical signal or an ultrasound signal, emanating from the object (3) in response to illuminating the object (3) with the first electromagnetic radiation, wherein the at least one electronic controller (3) is configured to derive, based on the detected response signal, a time measure characterizing a duration (time of flight) it takes for the first electromagnetic radiation to travel from the VCSEL (4') to the object (3) and, after reflection or conversion into an ultrasound wave by the object (3), back to the detector (14), and to derive positional information regarding a height position (z) and/or distance (d) of the at least one VCSEL (4') and/or handheld device (1) relative to the object (3).

2. The handheld device according to claim 1, wherein the at least one electronic controller is configured to control the at least one radiation source to emit the pulses of electromagnetic radiation dependent on the derived positional information.

3. The handheld device according to claim 2, wherein the at least one electronic controller is configured to control the at least one radiation source
- to emit the pulses of electromagnetic radiation only if the derived positional information is indicative that the handheld device, in particular the housing, is in contact with and/or close to the object, and/or
- to stop emitting the pulses of electromagnetic radiation if the derived positional information is indicative that the handheld device, in particular the housing, is not in contact with and/or not close to the object.

4. The handheld device according to claim 3, further comprising a contact sensor integrated in and/or provided on the housing and configured to detect contact information which is indicative whether the handheld device, in particular the housing, is in contact with the object or not, wherein the at least one electronic controller is configured to control the at least one radiation source
- to emit the pulses of electromagnetic radiation only if both the derived positional information and the contact information are indicative that the handheld device, in particular the housing, is in contact with or close to the object, respectively, and/or
- to stop emitting the pulses of electromagnetic radiation if either the derived positional information or the contact information is indicative that the handheld device, in particular the housing, is not in contact with or not close to the object, respectively.

5. The handheld device according to any one of the preceding claims, wherein the at least one electronic controller is configured to derive the positional information for
- each of a plurality of different lateral (x, y) positions of the handheld device while being moved laterally relative to the object, and/or
- each of a plurality of different height (z) positions of the handheld device while being moved relative, in particular towards and/or away from the object, and/or
- each of a plurality of different angular positions and/or directions relative to the object while being moved relative to the object.

6. The handheld device according to claim 5, wherein the at least one electronic controller is configured to
- reconstruct a plurality of first optoacoustic images, in particular two-dimensional optoacoustic images, based on the detection signals obtained for each of the plurality of different lateral positions, and
- reconstruct a second optoacoustic image, in particular a three-dimensional optoacoustic image, by stitching the first optoacoustic images using the positional information derived for each of the different lateral positions.

7. The handheld device according to any one of the preceding claims, wherein the at least one radiation source is and/or comprises the at least one vertical-cavity surface-emitting laser (VCSEL) and/or the detection unit comprises at least one micromachined ultrasonic transducer, in particular at least one capacitive micromachined ultrasonic transducer (CMUT) or piezoelectric micromachined ultrasonic transducer (PMUT).

8. The handheld device according to any one of the preceding claims, wherein the at least one electronic controller is configured to control the at least one VCSEL to emit pulses of electromagnetic radiation having a pulse repetition rate between 100 Hz and 10 kHz.

9. A handheld device (6) for ultrasound imaging of an object (3), the device (1) comprising a housing (2) configured to be grasped by an operator's hand to be positioned and/or moved relative to the object (3), at least one electronic controller (15), and an ultrasound sensor unit (7') which is integrated in and/or provided on the housing (2) and configured to transmit ultrasound (5') towards the object (3), to detect ultrasonic waves (8') emanating from the object (3), and to convert the detected ultrasonic waves (8') into detection signals,
**characterized by**
i) at least one VCSEL sensor unit (10) comprising a vertical-cavity surface-emitting laser (VCSEL), a laser resonator (12) and a photodiode (13) provided in and/or at the laser resonator (12) and being arranged and/or configured to: illuminate the object (3) with first electromagnetic radiation emerging from the laser resonator (12); allow second electromagnetic radiation, which has been reflected and/or scattered by the object (3) in response to illuminating the object (3) with the first electromagnetic radiation, to enter the laser resonator (12) and mix and/or interfere with the first electromagnetic radiation, whereby an interference signal is obtained; and detect the interference signal with the photodiode (13), wherein the at least one electronic controller (15) is configured to derive, based on the detected interference signal, positional information regarding a position and/or distance and/or movement of the at least one VCSEL sensor unit (10) and/or the handheld device (10) relative to the object (3), and/or
ii) at least one vertical-cavity surface-emitting laser (VCSEL) (4') configured to illuminate the object (3) with first electromagnetic radiation, a detector (7'; 14), in particular an optical or ultrasound detector, configured to, in particular incoherently, detect a response signal, in particular an optical signal or an ultrasound signal, emanating from the object (3) in response to illuminating the object (3) with the first electromagnetic radiation, wherein the at least one electronic controller (15) is configured to derive, based on the detected response signal, a time measure characterizing a duration (time of flight) it takes for the first electromagnetic radiation to travel from the VCSEL (4') to the object (3) and, after reflection or conversion into an ultrasound wave by the object (3), back to the detector (7'; 14), and to derive positional information regarding a height position (z) and/or distance of the at least one VCSEL (4') and/or handheld device (1) relative to the object (3).

10. A system for optoacoustic or ultrasound imaging of an object, the system comprising:
- a handheld device (1,6) according to at least one of the preceding claims, and
- a computer system (21, 22), in particular a mobile computer device and/or a computer workstation and/or a cloud computing system, located outside the handheld device (1, 6), in particular outside the housing (2) of the handheld device (1, 6), and being in a data communication with the at least one electronic controller (15), the computer system (21, 22) being configured to process the detection signals and/or to reconstruct an image based on the detection signals and/or to further process an image which has been reconstructed by the at least one electronic controller (15).
